# EUROPÄISCHE PATENTSCHRIFT

(11) **EP 1 096 961 B1**
(45) Veröffentlichungstag und Bekanntmachung des Hinweises auf die Patenterteilung: **26.03.2003**
(21) Anmeldenummer: 99947229.3
(22) Anmeldetag: 20.07.1999
(51) Int. Cl.: A61L 11/00, A61L 2/06

(54) **VERFAHREN UND VORRICHTUNG ZUR BEHANDLUNG VON KONTAMINIERTEN MATERIALIEN**
METHOD AND DEVICE FOR TREATING CONTAMINATED MATERIALS
PROCEDE ET DISPOSITIF POUR TRAITER DES MATIERES CONTAMINEES

(30) Priorität: 23.07.1998 DE 19833024
(43) Veröffentlichungstag der Anmeldung: 09.05.2001
(73) Patentinhaber: Göldner, Helmut, 31633 Leese (DE)
(72) Erfinder: Göldner, Helmut, 31633 Leese (DE)
(74) Vertreter: Braun, Dieter, Dipl.-Ing.
(86) Internationale Anmeldenummer: DE9902212
(87) Internationale Veröffentlichungsnummer: WO00004935

(56) Entgegenhaltungen:
- EP-A- 0 672 426
- WO-A-98/48853
- DE-A- 2 952 544
- DE-A- 4 138 939
- DE-C- 3 938 546
- DE-U- 9 213 599
- US-A- 3 464 342
- DATABASE WPI Section Ch, Week 199932 Derwent Publications Ltd., London, GB; Class D14, AN 1999-374391 XP002123068 & JP 11 137644 A (THERMAL KK), 25. Mai 1999 (1999-05-25)

## Beschreibung

Die Erfindung bezieht sich auf ein Verfahren zur Behandlung von kontaminierten, insbesondere infizierten Materialien, wobei diese über eine Eingabeeinheit einem sich in einer Behandlungskammer erstreckenden Transportsystem zugeführt, aufgeheizt und dort desinfiziert bzw. sterilisiert, sowie an einem Auswurf ausgetragen werden. Ferner betrifft die Erfindung eine Vorrichtung für obiges Verfahren, bei der im wesentlichen die gesamte Behandlungskammer in Förderrichtung aufwärts gerichtet geneigt ist.

Durch die DE 39 38 546 C2 ist bereits eine Hochtemperatur-Desinfektionsanlage für krankenhausspezifische Abfälle bekannt geworden, bei der die Abfälle über einen Einlaßtrichter zwei durch eine druckdichte mechanische Zwischenschleuse getrennten Schneckenstreckenabschnitten zugeführt werden. Dabei wird im ersten Schneckenstreckenabschnitt durch Wärmeeinleitung ein Dampfdruck einstellbar erzeugt, während im zweiten Schneckenstreckenabschnitt ein Unterdruck erzeugt wird, um das Gut durch Absaugen der Dämpfe zu entfeuchten. Bei dieser bekannten Anlage ist es von Nachteil, daß die zwei Schneckenabschnitte durch eine mechanische Druckschleuse getrennt sind, die aufwendig ist und natürlich auch eine potentielle Fehlerquelle darstellt. Hinzu kommt, daß die Schneckenstreckenabschnitte in der horiziontalen Ebene angeordnet sind, so daß kontaminierte Flüssigkeit unbemerkt durch die Anlage fließen und den Desinfektionsprozeß unbehandelt bzw. unzulänglich behandelt passieren kann. Es ist hier keineswegs sichergestellt, daß unter allen Bedingungen eine sichere Desinfektion bzw. insbesondere auch Sterilisation der Abfälle erfolgt.

Aus der DE 92 13 599 U1 ist eine Vorrichtung zur Regeneration und Sterilisation von Erde bekannt. Bei dieser Vorrichtung wird die Erde durch eine schräggestellte Behandlungskammer geleitet und dabei mit Wasserdampf beaufschlagt. Nachteilig bei dieser Vorrichtung ist jedoch, daß nicht zwei verschiedene Behandlungszonen vorgesehen sind, die zur Benetzung bzw. zur Desinfektion oder Sterilisation des zu behandelnden Materials dienen. Vielmehr finden Desinfektion oder Sterilisation über den gesamten Bereich der Behandlungskammer statt. Ferner kann bei der Vorrichtung kein Druckaufbau stattfinden, da sie kein geschlossenes System darstellt.

Durch die DE 41 38 939 A ist auch bereits eine Vorrichtung und ein Verfahren zum Sterilisieren und Desinfizieren von kontaminiertem Krankenhausmüll bekannt, bei dem der Müll zunächst zerkleinert und das so gewonnene feuchte Granulat anschließend einer in einer schräg gestellten Behandlungskammer angeordneten Desinfektionsschnecke zugeführt wird, wobei drei Bereiche zur Behandlung vorgesehen sind. Das Granulat wird zunächst im unteren Bereicht getrocknet und anschließend im nächsten, höher gelegenen Abschnitt der Desinfektionsschnecke mit strömendem Dampf verwirbelt und desinfiziert, um schließlich im oberen Teil der Desinfektionsschnecke erneut getrocknet und über eine Förderanlage einem Container zugeführt zu werden.

Ferner sind in der DE 44 09 391 A1 ein Verfahren und eine Vorrichtung zum Dekontaminieren von Schüttgut beschrieben. Auch bei diesem Stand der Technik sind jedoch nicht zwei verschiedene Behandlungszonen zur Benetzung bzw. zur Dekontaminierung des Materials vorgesehen. Zudem ist ebenfalls kein zum Druckaufbau geeignetes geschlossenes System vorgesehen.

Der Erfindung liegt die Aufgabe zugrunde, ein Verfahren und eine kompakte Vorrichtung zu schaffen, die unter Einsatz einfacher technischer Mittel verschiedene Behandlungszonen für Chargenbetrieb, ein- und mehrstufige Prozesse und kontinuierlichen Betrieb ermöglichen, wobei stets eine sichere Desinfektion bzw. auch Sterilisation kontaminierter Materialien gewährleistet ist.

Diese Aufgabe wird erfindungsgemäß durch die Merkmale des Anspruchs 1 sowie des Anspruchs 8 gelöst. Die weitere Ausgestaltung der Erfindung ist den Unteransprüchen zu entnehmen.

Bei dem erfindungsgemäßen Verfahren wird eine erste Behandlungszone zur Benetzung des infizierten Materials geschaffen, indem in einem ersten Bereich der Behandtungskammer durch eine in Förderrichtung aufwärts gerichtete Neigung, im wesentlichen der gesamten Behandlungskammer, ein Flüssigkeitsreservoir mittels in dem infizierten Material vorhandener Flüssigkelt und/oder externer Zugabe von Wasser erzeugt wird und das Flüssigkeitsreservoir auf eine Temperatur, die geringer als die Siedetemperatur von Wasser ist, erwärmt wird, wobei der erste Bereich benachbart zu dem tieferliegenden Ende der Behandlungskammer ist. Ferner wird eine zweite Behandlungszone zur Desinfektion bzw. Sterilisation geschaffen, indem ein zweiter Bereich, der sich von dem ersten Bereich bis zu dem höherliegenden Ende der Behandlungskammer erstreckt, zumindest teilweise auf eine Temperatur, die höher als die Siedetemperatur von Wasser ist, erwärmt und in dem zweiten Bereich der zur Desinfektion bzw. Sterilisation erforderliche Dampfdruck aufgebaut wird.

Auf diese Weise ist erreicht, daß kontaminierte Flüssigkeit sich stets in einem definierten Bereich der Behandlungskammer sammelt und damit nicht unbemerkt an eine ungewünschte Stelle einer verwendeten Vorrichtung fließen kann. Darüber hinaus sind bei dem Verfahren mindestens zwei Behandlungszonen geschaffen, nämlich eine erste, in der das infizierte Material innerhalb des Flüssigkeitsreservoirs benetzt wird, und eine zweite, in der die Temperatur und der Dampfdruck gegeben sind, die zur Desinfektion bzw.

Sterilisation erforderlich sind. Das Verfahren erlaubt einen kontinuierlichen bzw. quasikontinuierlichen Desinfektions- bzw. Sterilisationsprozeß, wobei aufgrund kurzer Wege die Verweilzeiten in den verschiedenen Behandlungszonen optimal gewährleistet werden können.

Es kann vorgesehen sein, den zweiten Bereich der Behandlungskammer in Abschnitte mit jeweils unterschiedlicher Temperatur zu unterteilen. So kann beispielsweise ein Abschnitt, der unmittelbar an den ersten Bereich angrenzt, eine geringere Temperatur als der folgende Abschnitt aufweisen und damit als Übergangsabschnitt dienen. In diesem Übergangsabschnitt kann z. B. Wasserdampf zugeführt werden, um den gewünschten Dampfdruck in dem zweiten Bereich zu erhalten, in dem die zweite Stufe des Desinfektions- bzw. Sterilisationsprozesses stattfindet. Ferner können insbesondere in diesem Übergangsabschnitt verschiedene Meßvorgänge zur Bestimmung von Prozeßparametern durchgeführt werden.

In dem ersten Bereich können Mittel zur Zugabe von Wasser in flüssiger Form vorgesehen sein, um das Niveau des Flüssigkeitsreservoirs auf vorgegebener Höhe zu halten. Der maximale Füllstand des Flüssigkeitsreservoirs wird vorzugsweise über einen Überlauf reguliert. Dabei ist es zweckmäßig, wenn in den Überlauf gelangte Flüssigkeit in das Flüssigkeitsreservoir zurückgeführt werden kann, um sie zu einem späteren Zeitpunkt ebenfalls zu desinfizieren oder sterilisieren. Alternativ kann vorgesehen sein, die Flüssigkeit in einem separaten Prozeß zu desinfizieren bzw. sterilisieren.

Es kann vorgesehen sein, daß das zu behandelnde Material in kleineren Portionen zugeführt wird, wobei die Zufuhr und der Austrag über Schieber und/oder Druckschleusen der Eingabeeinheit und des Auswurfs erfolgt. Dabei befinden sich die Portionen in jeweils unterschiedlichen Behandlungsstufen. Durch die Schleusen kann sichergestellt werden, daß bei der Zufuhr bzw. dem Austrag von Chargen kein nennenswerter Druckverlust erfolgt, sich die technischen Prozeßparameter also nicht verändern. Es kann aber auch eine geringe vorübergehende Druckschwankung beabsichtigt sein, um durch solch ein "Atmen" die Effektivität des Desinfektions- bzw. Sterilisationsprozesses positiv zu beeinflussen.

Das bei dem Verfahren verwendete Transportsystem weist vorzugsweise eine Förderschnecke auf.

Bei dem Verfahren kann der gewünschte Sattdampf in dem zweiten Bereich der Behandlungskammer allein dadurch erzeugt werden, daß zu behandelndes Material aus dem Flüssigkeitsreservoir herausbefördert wird und somit benetzt in den zweiten Bereich der Behandlungskammer gelangt, in dem das auf der Oberfläche im Abfall befindliche Wasser verdampft. In der Regel reicht diese Eigenfeuchtigkeit des zu behandelnden Materials aus, um den nötigen Dampfdruck zu erreichen. Wenn der so erhaltene Dampfdruck jedoch nicht ausreichend ist, kann zusätzlich Wasserdampf zugeführt werden.

Die erfindungsgemäße Vorrichtung ist dadurch gekennzeichnet, daß die Behandlungskammer eine erste Heizzone aufweist, die benachbart zu dem tieferliegenden Ende der Behandlungskammer ist und zur Erzeugung einer Temperatur unterhalb der Siedetemperatur von Wasser ausgelegt ist, und ferner eine zweite Heizzone, die sich zwischen der ersten Heizzone und dem höherliegenden Ende der Behandlungskammer erstreckt und zur Erzeugung einer Temperatur oberhalb der Siedetemperatur von Wasser und zum Aufbau des zur Desinfektion bzw. Sterilisation erforderlichen Dampfdrucks ausgelegt ist.

Durch die Schrägstellung der Behandlungskammer wird erreicht, daß sich Flüssigkeit, die sich in der Behandlungskammer zugeführten kontaminierten Materialien befindet und beispielsweise durch Einwirkung eines vorgeschalteten Zerkleinerers freigesetzt worden ist, in der ersten Heizzone sammelt. Dadurch ist zum einen gewährleistet, daß kontaminierte Flüssigkeit nicht in unerwünschte Bereiche der Behandlungskammer gelangt. Zum anderen ist durch die Ansammlung der kontaminierten Flüssigkeit und ggfs. durch externe Zugabe von Wasser ein Flüssigkeitsreservoir erzeugt, das zur Benetzung der zu behandelnden Materialien verwendet werden kann. Die erste Heizzone ist so ausgelegt, daß das Flüssigkeitsreservoir auf eine Temperatur aufgeheizt werden kann, die geringfügig unterhalb der Siedetemperatur von Wasser liegt, d. h. bei Atmosphärendruck unter 100° C. Dadurch, daß die Temperatur unterhalb der jeweiligen Siedetemperatur liegt, werden eine hohe Verdampfungsrate des Wassers und unerwünschte Verkrustungen an der Wandung der Behandlungskammer oder an dem Transportsystem vermieden. Außerdem wird dadurch, daß die Siedetemperatur nicht erreicht wird, verhindert, daß Dämpfe und mögliche Gerüche bei Öffnung der Eingabeeinheit austreten können. Die erfindungsgemäße Vorrichtung ermöglicht bei geringem Platzbedarf eine Erzeugung mehrerer Temperatur- bzw. Behandlungszonen. Aufgrund der technischen Mittel ist eine sichere hermetische Abschirmung und damit eine sichere reproduzierbare Prozeßführung gewährleistet, bei der eine sehr wirtschaftliche Desinfektion bzw. Sterilisation durchgeführt werden kann.

Die erste Heizzone kann beispielsweise Mittel zur Zugabe von flüssigem Wasser aufweisen, um eine Niveauregelung der Flüssigkeit in dem Flüssigkeitsreservoir durchführen zu können. In der zweiten Heizzone findet die zweite Stufe des Desinfektions- bzw. Sterilisationsprozesses statt. Die zweite Heizzone kann Mittel zur Einführung von Wasser in flüssiger Form und von Wasserdampf aufweisen, um auch für den Fall, daß die Eigenfeuchtigkeit des zu behandelnden Materials nicht ausreicht, Sattdampf erzeugen zu können. Außerdem können in der zweiten Heizzone auch Mittel zum Anschließen von Meßgeräten verschiedener Art, insbesondere Temperatur-, Feuchte- und Druckmeßgeräten, vorgesehen sein und auch Mittel zur Zugabe von Zuschlagstoffen. Im Rahmen der Erfindung kann vorgesehen sein, daß die zweite Heizzone in weitere Heizabschnitte zur Erzeugung weiterer Temperaturen unterteilt ist. So kann beispielsweise ein unmittelbar an die erste Heizzone angrenzender Abschnitt der zweiten Heizzone in bezug auf die Temperatur einen Übergang von der ersten Heizzone zu einem Abschnitt der zweiten Heizzone darstellen, in dem die zur Desinfektion bzw. Sterilisation benötigte Temperatur herrscht.

Es kann ferner vorgesehen sein, daß die Behandlungskammer einen Überlauf zur Regulierung des Flüssigkeitsreservoirs aufweist. Dieser Überlauf mündet vorzugsweise in einen druckdichten Auffangbehälter, der durch eine Rückführleitung wiederum mit der Behandlungskammer verbunden ist. Vorzugsweise sind der Überlauf, der Auffangbehälter und die Rückführleitung so ausgelegt, daß in ihnen derselbe Druck wie in der Behandlungskammer herrscht. Auf diese Weise ist es möglich, durch ein einfaches Pumpsystem Flüssigkeit aus dem Auffangbehälter in die Behandlungskammer zu pumpen, wenn dort der Flüssigkeitspegel erhöht werden soll. Es kann auch eine zusätzliche Hochtemperaturdesinfektions- bzw. Hochtemperatursterilisationseinheit vorgesehen sein, in der die Flüssigkeit aus dem Auffangbehälter behandelt werden kann. Dabei kann auch der Auffangbehälter selbst als Autoklav ausgelegt sein.

Zur Erzeugung der jeweiligen Temperaturen in den verschiedenen Heizzonen bzw. Heizabschnitten kann eine Reihe von Heizmitteln eingesetzt werden. So kann beispielsweise die Wandung der Behandlungskammer mit Heizmitteln versehen sein. Diese können in einem mit Wärmeträgeröl versehenen Doppelmantel bestehen. Dabei wird das Wärmeträgeröl durch einen Aufheizblock erwärmt.

Es kann auch vorgesehen sein, daß in der Behandlungskammer und/oder dem Transportsystem Mikrowellenenergie definiert in feuchtes Material einleitbar ist, um das Material auf die gewünschte Temperatur zu erwärmen.

Das Transportsystem weist vorzugsweise eine Förderschnecke auf. Diese kann reversierbar ausgelegt sein, um ggfs. eine Druckentlastung bei einem Beförderungsengpaß bewirken zu können. Es ist vorteilhaft, wenn die Förderschnecke nur an einem Ende ein Lager aufweist und auf Verschleißschienen ruht.

Es ist sehr zweckmäßig, wenn in der Eingabeeinheit ein Zerkleinerer angeordnet ist, der insbesondere zur Zerkleinerung von kontaminierten Krankenhausabfällen vorteilhaft ist.

Zur Kapazitätssteigerung kann eine Anlage vorgesehen sein, die mehrere der oben beschriebenen Vorrichtungen und eine Zerkleinerereinheit aufweist, wobei die Vorrichtungen parallel so angeordnet sind, daß sie von der Zerkleinerereinheit zeitgleich und/oder zeitversetzt beschickt werden können. Auf diese Weise kann auch dann, wenn die einzelnen Vorrichtungen im sogenannten Chargenbetrieb eingesetzt werden, eine quasi-kontinuierliche Desinfektion bzw. Sterilisation durchgeführt werden.

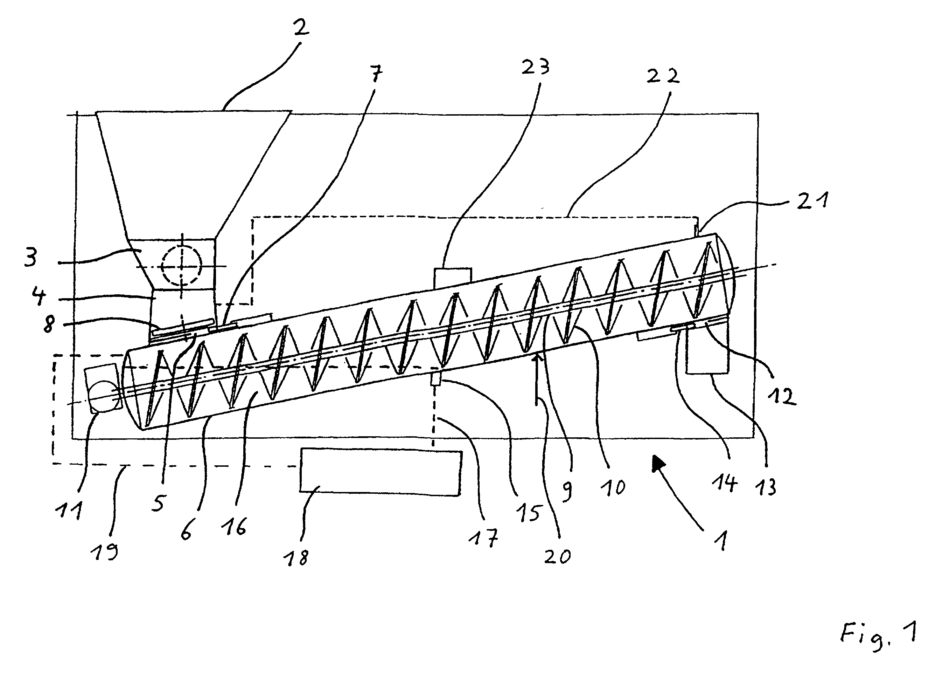

Im folgenden wird die erfindungsgemäße Vorrichtung anhand eines Ausführungsbeispiels näher erläutert, wobei auf die einzige Figur Bezug genommen wird. Die Figur zeigt schematisch eine erfindungsgemäße Vorrichtung.

In der Figur ist mit 1 eine Vorrichtung zur Behandlung von kontaminierten, insbesondere infizierten Materialien, bezeichnet. Die Vorrichtung 1 weist als Bestandteil einer Eingabeeinheit einen Einwurftrichter 2 auf, unter dem ein Zerkleinerer 3 angeordnet ist. Von dem Zerkleinerer 3 führt ein Unterfalltrichter 4 zu einem Einlaß 5 einer rohrförmigen Behandlungskammer 6. Der Einlaß 5 ist durch einen Schieber 7 verschließbar. Anstelle des Schiebers 7 oder zusätzlich zum Schieber 7 könnte auch eine Schleuse vorgesehen sein. Oberhalb des Einlasses 5 ist ein Dosierer 8 angeordnet.

In der Behandlungskammer 6 erstreckt sich eine Förderschnecke 9, die eine Transportspirale 10 aufweist. Die Förderschnecke 9 wird über einen Antrieb 11 angetrieben. Die Behandlungskammer 6 ist in Förderrichtung der Förderschnecke 9 schräg nach oben gerichtet, beispielsweise mit einem Winkel zur Horizontalen von etwa 10° bis 40°. An dem oberen Ende der Behandlungskammer 6 befindet sich ein Auswurf 12 mit einem Auswurfschacht 13. Auch der Auswurf 12 ist mit einem Schieber 14 versehen. Auch hier könnte natürlich anstelle des Schiebers 14 oder zusätzlich zum Schieber 14 eine Schleuse vorgesehen sein.

Ein Überlauf 15 für Flüssigkeit 16, die sich in einem unteren Bereich der Behandlungskammer 6 ansammeln kann, ist an der Unterseite der Behandlungskammer 6 angeordnet. Der Überlauf 15 ist über eine Rohrleitung 17 mit einem Auffangbehälter 18 verbunden. Der Auffangbehälter 18 ist über eine weitere Rohrleitung 19 mit der Behandlungskammer venbunden, wobei die Rohrleitung 19 oberhalb des Maximalpegels der Flüssigkeit 16 in das untere Ende der Behandlungskammer 6 mündet.

Die Vorrichtung 1 weist zwei Heizzonen auf, in denen unterschiedliche Temperaturen erzeugt werden. Die erste Heizzone erstreckt sich von dem unteren Ende der Behandlungskammer 6 bis zu dem Überlauf 15. Die zweite Heizzone schließt sich unmittelbar an die erste Heizzone an und erstreckt sich bis zu dem oberen Ende der Behandlungskammer 6. Die Heizmittel der beiden Heizzonen sind zur Vereinfachung der Figur nicht dargestellt. Sie können z. B. in einem mit Wärmeträgeröl gefüllten Doppelmantel der Behandlungskammer 6 bestehen, wobei der Doppelmantel entsprechend der beiden Heizzonen zwei Kammern aufweist. Dabei wird das Wärmeträgeröl beispielsweise durch zwei getrennte Aufheizblöcke aufgeheizt. Auch der Einsatz von Wärmetauschern ist möglich.

An dem oberen Ende der Behandlungskammer 6 befindet sich ein Entlüftungsventil 21, das über eine Rohrleitung 22 mit dem Unterfalltrichter 4 verbunden ist Ferner sind in der zweiten Heizzone Einlaßmittel 23 zur Zugabe von Wasserdampf vorgesehen.

Der Dosierer 8 ist über dem Einlaß 5 angeordnet, der als Schlitz in einer Bodenplatte geformt und durch den Schieber 7 verschließbar ist.

Zur Behandlung von kontaminierten Materialien wird der Vorrichtung 1 über den Einwurftrichter 2 das zu behandelnde Material zugeführt. Der Zerkleinerer 3 zerkleinert das Material auf eine Größe von beispielsweise ungefähr 10 x 20 mm im Querschnitt, wobei bei Krankenhausabfällen Hohlkörper, wie Spritzen, zerstört werden. Das Material wird dann über den Unterfalltrichter 4 dem Dosierer 8 zugeleitet. Der Dosierer 8 sorgt dafür, daß das Material nach Zerkleinerung in den Einlaß 5 gelangt, ohne daß eine Brückenbildung innerhalb des Einlasses 5 auftritt. Auch die kegelstumpfförmige Form des Unterfalltrichters 4 trägt dazu bei, daß eine Brückenbildung verhindert wird.

In der Behandlungskammer 6 gelangt das zu behandelnde Material in das Flüssigkeitsreservoir 16. Der durch den Zerkteinerer 3 freigesetzte Flüssigkeitsanteil des zu behandelnden Materials trägt zu dem Flüssigkeitsreservoir 16 bei, dessen Pegel durch einen nicht gezeigten Flüssigkeitssensor gemessen und ggfs. durch Zuführung von Flüssigkeit aus dem Auffangbehälter 18 oder durch Zugabe von flüssigem Wasser auf den Sollwert erhöht werden kann. Das Flüssigkeitsreservoir 16 weist eine Temperatur auf, die geringfügig tiefer als die jeweilige Siedetemperatur von Wasser ist. Auf diese Weise werden Verkrustungen an der Wandung der Behandlungskammer 6 oder an der Förderschnecke 9 vermieden. In dem Flüssigkeitsreservoir 16 wird das zu behandelnde Material durchtränkt. Durch die Förderschnecke 9 wird das Material anschließend in die zweite Heizzone transportiert. In dieser Heizzone herrscht im wesentlichen, d.h. bis auf einen von dem Überlauf 15 bis etwa zu der durch den Pfeil 20 angedeuteten Höhe der Behandlungskammer 6 sich erstreckenden Übergangsabschnitt der zweiten Heizzone, eine Temperatur, die oberhalb der Siedetemperatur liegt, so daß das Wasser des benetzten Materials verdampft und sich ein entsprechender Dampfdruck aufbaut. Die Prozeßbedingungen werden so eingestellt, daß sich das Material für eine Desinfektion beispielsweise auf eine Temperatur von mehr als 100 °C und für eine Sterilisation auf eine Temperatur von mindestens 121 °C erwärmt. Dazu wird Sattdampf erzeugt, der, wenn die Eigenfeuchtigkeit des Materials nicht ausreicht, auch dadurch erzeugt werden kann, daß Wasserdampf über die Einlaßmittel 23 der Behandlungskammer 6 zugeführt wird. In der zweiten Heizzone findet der eigentliche Desinfektions- bzw. Sterilisationsvorgang statt, der sich beispielsweise über einen Zeitraum von mindestens 15 Minuten erstrecken kann. Nach Beendigung des Behandlungsprozesses wird zunächst das Entlüftungsventil 21 geöffnet, um den Dampfdruck abzulassen. Dabei wird der Wasserdampf dem Unterfalltrichter 4 zugeführt, in dem sich bereits weiteres zu behandelndes Material befinden kann, das durch den Dampf vorgeheizt wird. Gleichzeitig findet eine weitere Entfeuchtung des behandelten Materials statt, indem Wasser, das sich auf der Oberfläche des Materials befindet oder kapillar in diesem gebunden ist, aufgrund des Druckabbaus unter Ausnutzung der fühlbaren Wärme des Materials verdampft, während sich die Temperatur des Wassers und des Materials der Siedetemperatur bei Normaldruck annähert. Anschließend wird der Auswurf 12 durch den Schieber 14 geöffnet, um das behandelte Material auszutragen. Vorzugsweise wird gleichzeitig der Einlaß 5 geöffnet, um entsprechend der ausgeworfenen Materialmenge weiteres zu behandelndes Material in die Behandlungskammer 6 einzubringen.

Wenn anstelle der Schieber 7 und 14 oder zusätzlich zu den Schiebern Schleusen vorgesehen sind, ist auch ein kontinuierlicher Desinfektions- bzw. Sterilisationsprozess möglich.

Mit der erfindungsgemäßen Vorrichtung können kontaminierte Materialien, bei denen es sich vorzugsweise um krankenhausspezifische Abfälle, aber z. B. auch um Klärschlamm, kontaminierte Böden sowie Lebensmittel, wie Getreide und Gewürze, handeln kann, sicher desinfiziert bzw. sterilisiert werden. Durch die kompakte Gestaltung der Vorrichtung ist eine wenig aufwendige und sichere Behandlung der Materialien und zudem die Herstellung kompakter, leistungsfähiger mobiler Anlagen möglich.

## Patentansprüche

1. Verfahren zur Behandlung von kontaminierten, insbesondere von infizierten Materialien, bei dem diese über eine Eingabeeinheit (2, 3, 4) einem sich in einer Behandlungskammer (6) erstreckenden Transportsystem (9) zugeführt, aufgeheizt und dort desinfiziert bzw. sterilisiert sowie an einem Auswurf (12) ausgetragen werden, **dadurch gekennzeichnet, daß**
eine erste Behandlungszone zur Benetzung des infizierten Materials geschaffen wird, indem in einem ersten Bereich der Behandlungskammer (6) durch eine in Förderrichtung aufwärts gerichtete Neigung im wesentlichen der gesamten Behandlungskammer (6) ein Flüssigkeitsreservoir (16) mittels in dem infizierten Material vorhandener Flüssigkeit und/oder externer Zugabe von Wasser erzeugt wird und das Flüssigkeitsreservoir auf eine Temperatur, die geringer als die Siedetemperatur von Wasser ist, erwärmt wird, wobei der erste Bereich benachbart zu dem tieferliegenden Ende der Behandlungskammer ist,
und eine zweite Behandlungszone zur Desinfektion bzw. Sterilisation geschaffen wird, indem ein zweiter Bereich, der sich von dem ersten Bereich bis zu dem höherliegenden Ende der Behandlungskammer (6) erstreckt, zumindest teilweise auf eine Temperatur, die höher als die Siedetemperatur von Wasser ist, erwärmt und in dem zweiten Bereich der zur Desinfektion bzw. Sterilisation erforderliche Dampfdruck aufgebaut wird.

2. Verfahren nach Anspruch 1, **dadurch gekennzeichnet, daß** der zweite Bereich in Abschnitte mit unterschiedlichen Temperaturen unterteilt wird.

3. Verfahren nach Anspruch 2, **dadurch gekennzeichnet, daß** in dem zweiten Bereich der gewünschte Dampfdruck durch Verdampfung von Eigenfeuchtigkeit zu behandelnden Materials und/oder durch Zuführung von Wasser in flüssiger Form und/oder Wasserdampf erzeugt wird.

4. Verfahren nach einem der vorhergehenden Ansprüche, **dadurch gekennzeichnet, daß** der Füllstand des Flüssigkeitsreservoirs (16) mittels eines Überlaufs (15) reguliert wird.

5. Verfahren nach Anspruch 4, **dadurch gekennzeichnet, daß** Flüssigkeit aus dem Überlauf (15) in das Flüssigkeitsreservoir (16) zurückgeführt wird.

6. Verfahren nach einem der vorhergehenden Ansprüche, **dadurch gekennzeichnet, daß** das zu behandelnde Material in Portionen zugeführt wird, die sich zur selben Zeit in der Behandlungskammer (6) befinden, wobei die Zufuhr und der Austrag über Schieber oder Schleusen der Eingabeeinheit (2, 3, 4) und des Auswurfs (12) erfolgt.

7. Verfahren nach einem der vorhergehenden Ansprüche, **dadurch gekennzeichnet, daß** das Transportsystem eine Förderschnecke (9) aufweist.

8. Vorrichtung zur Behandlung von kontaminierten, insbesondere von infizierten Materialien, bei der diese über eine Eingabeeinheit (2, 3, 4) einem sich in einer Behandlungskammer (6) erstreckenden Transportsystem (9) zugeführt, aufgeheizt und dort desinfiziert bzw. sterilisiert, sowie an einem Auswurf (12) ausgetragen werden, wobei im wesentlichen die gesamte Behandlungskammer (6) in Förderrichtung aufwärts gerichtet geneigt ist und die Behandlungskammer (6) eine erste Heizzone, die benachbart zu dem tieferliegenden Ende der Behandlungskammer angeordnet ist, und eine zweite Heizzone aufweist, die sich zwischen der ersten Heizzone und dem höherliegenden Ende der Behandlungskammer (6) erstreckt und zur Erzeugung einer Temperatur zum Aufbau des zur Desinfektion bzw. Sterilisation erforderlichen Dampfdrucks ausgelegt ist, **dadurch gekennzeichnet, daß** die erste Heizzone mit Mitteln (23, Flüssigkeitsreservoir 16) zur Benetzung der einzufüllenden Materialien mit Flüssigkeit ausgestattet ist und zur Erzeugung einer Temperatur unterhalb der Siedetemperatur von Wasser ausgelegt ist und die zweite Heizzone zur Erzeugung einer Temperatur oberhalb der Siedetemperatur von Wasser ausgelegt ist.

9. Vorrichtung nach Anspruch 8, **dadurch gekennzeichnet, daß** die zweite Heizzone in weitere Helzabschnitte zur Erzeugung weiterer Temperaturen unterteilt ist

10. Vorrichtung nach Anspruch 8 oder 9, **dadurch gekennzeichnet, daß** die zweite Heizzone Mittel (23) zur Einführung von Wasserdampf aufweist.

11. Vorrichtung nach Anspruch 8 bis 10, **dadurch gekennzeichnet, daß** die erste Heizzone Mittel (23) zur Zugabe von flüssigem Wasser aulweist.

12. Vorrichtung nach einem der Ansprüche 8 bis 11, **dadurch gekennzeichnet, daß** die Behandlungskammer (6) einen Überiauf (15) zur Regulierung eines Flüssigkeitsreservoirs (16) aufweist.

13. Vorrichtung nach Anspruch 12, **dadurch gekennzeichnet, daß** der Überlauf (15) in einen Auffangbehälter (18) mündet und dieser durch eine Rückführleitung (19) mit der Behandlungskammer (6) verbunden ist.

14. Vorrichtung nach Anspruch 13, **dadurch gekennzeichnet, daß** Überlauf (15), Auffangbehälter (18) und Rückführleitung (19) so ausgelegt sind, daß in ihnen derselbe Druck herrscht, wie in der Behandlungskammer (6).

15. Vorrichtung nach einem der Ansprüche 8 bis 14, **dadurch gekennzeichnet, daß** die Wandung der Behandlungskammer (6) mit Heizmitteln versehen ist.

16. Vorrichtung nach einem der Ansprüche 8 bis 15, **dadurch gekennzeichnet, daß** das Transportsystem (9) mit Heizmitteln versehen ist.

17. Vorrichtung nach einem der Ansprüche 8 bis 16, **dadurch gekennzeichnet, daß** in die Behandlungskammer (6) und/oder das Transportsystem (9) Mikrowellenenergie definiert einleitbar ist.

18. Vorrichtung nach einem der Ansprüche 8 bis 17, **dadurch gekennzeichnet, daß** das Transportsystem eine Förderschnecke (9) aufweist.

19. Vorrichtung nach Anspruch 18, **dadurch gekennzeichnet, daß** die Förderschnecke (9) nur an einem Ende ein Lager aufweist und auf Verschleißschienen ruht.

20. Vorrichtung nach einem der Ansprüche 8 bis 19, **dadurch gekennzeichnet, daß** in der Eingabeeinheit ein Zerkleinerer (3) angeordnet ist.

21. Vorrichtung nach einem der Ansprüche 8 bis 20, **dadurch gekennzeichnet, daß** die Eingabeeinheit (2, 3, 4) und der Auswurf (12) mittels Schieber (7, 14) und/oder Schleusen absperrbar sind.

22. Anlage, die mehrere Vorrichtungen nach einem der Ansprüche 8 bis 21 und eine Zerkleinerereinheit aufweist, wobei die Vorrichtungen parallel so angeordnet sind, daß sie von der Zerkleinerereinheit zeitgleich und/oder zeitversetzt beschickt werden können.

## Claims

1. Method for treating contaminated materials, in particular infected materials, in which the infected materials are fed by means of an input unit (2, 3, 4) to a conveyor system (9) extending in a treatment chamber (6) where they are heated, disinfected and sterilised and are discharged via an ejector (12), **characterised in that** a first treatment zone is provided for wetting the infected material **in that** a liquid reservoir (16) is created in a first region of the treatment chamber (6) by inclining substantially the entire treatment chamber (6) upwardly in the conveying direction by means of liquid provided in the infected material and/or external addition of water and the liquid reservoir is heated to a temperature which is below the boiling point of water, the first region being adjacent to the lower end of the treatment chamber, and a second treatment zone is provided for disinfection and sterilisation **in that** a second region extending from the first region to the higher end of the treatment chamber (6) is at least partially heated to a temperature above the boiling point of water and the vapour pressure required for disinfection and sterilisation is built up in the second region.

2. Method according to claim 1, **characterised in that** the second region is divided into sections with different temperatures.

3. Method according to claim 2, **characterised in that** the desired vapour pressure is created in the second region by evaporation of the inherent moisture in the material to be treated and/or by the supply of water in liquid and/or vapour form.

4. Method according to any one of the preceding claims, **characterised in that** the fullness of the liquid reservoir (16) is regulated by means of an overflow (15).

5. Method according to claim 4, **characterised in that** liquid from the overflow (15) is returned to the liquid reservoir (16).

6. Method according to any one of the preceding claims, **characterised in that** the material to be treated is supplied in portions which are located in the treatment chamber (6) at the same time, supply and discharge being effected via slide valves or sluices of the input unit (2, 3, 4) and the ejector (12).

7. Method according to any one of the preceding claims, **characterised in that** the conveyor system comprises a conveying screw (9).

8. Device for treating contaminated materials, in particular infected materials, in which the infected materials are fed by means of an input unit (2, 3, 4) to a conveyor system (9) extending in a treatment chamber (6) where they are heated, disinfected and sterilised and are discharged via an ejector (12), wherein substantially the entire treatment chamber (6) is inclined upwardly in the conveying direction and the treatment chamber (6) comprises a first heating zone arranged adjacent to the lower end of the treatment chamber and a second heating zone extending between the first heating zone and the higher end of the treatment chamber (6) and being constructed to produce a temperature for building up the vapour pressure required for disinfection and sterilisation, **characterised in that** the first heating zone is equipped with means (23, liquid reservoir 16) for wetting the materials to be introduced with liquid and is constructed for producing a temperature below the boiling point of water and the second heating zone is constructed for producing a temperature above the boiling point of water.

9. Device according to claim 8, **characterised in that** the second heating zone is divided into further heating sections for producing further temperatures.

10. Device according to claim 8 or 9, **characterised in that** the second heating zone comprises means (23) for introducing water vapour.

11. Device according to claims 8 to 10, **characterised in that** the first heating zone comprises means (23) for adding liquid water.

12. Device according to any one of claims 8 to 11, **characterised in that** the treatment chamber (6) comprises an overflow (15) for regulating a liquid reservoir (16).

13. Device according to claim 12, chaxactezised in that the overflow (15) opens into a receiver (18) and the receiver (18) is connected to the treatment chamber (6) by a return line (19).

14. Device according to claim 13, **characterised in that** the overflow (15), receiver (18) and return line (19) are constructed in such a way that they are subjected to the same pressure as the treatment chamber (6).

15. Device according to any one of claims 8 to 14, **characterised in that** the wall of the treatment chamber (6) is provided with heating means.

16. Device according to any one of claims 8 to 15, **characterised in that** the conveyor system (9) is provided with heating means.

17. Device according to any one of claims 8 to 16, **characterised in that** microwave energy may be introduced into the treatment chamber (6) and/or the conveyor system (9) in a defined manner.

18. Device according to any one of claims 8 to 17, **characterised in that** the conveyor system comprises a conveying screw (9).

19. Device according to claim 18, **characterised in that** the conveying screw (9) comprises a bearing only at one end and rests on wearing rails.

20. Device according to any one of claims 8 to 19, **characterised in that** a comminuter (3) is arranged in the input unit.

21. Device according to any one of claims 8 to 20, **characterised in that** the input unit (2, 3, 4) and ejector (12) may be blocked by means of slide valves (7, 14) and/or sluices.

22. Installation comprising a plurality of devices according to any one of claims 8 to 21 and a comminuter unit, wherein the devices are arranged in parallel in such a way that they may be loaded simultaneously and/or in a time-staggered manner by the comminuter unit.

## Revendications

1. Procédé pour le traitement de matériels contaminés, en particulier infectés, dans lequel ceux-ci sont amenés par l'intermédiaire d'une unité d'entrée (2, 3, 4) à un système de transport (9) s'étendant dans une chambre de traitement (6), chauffés et, là, désinfectés ou stérilisés, ainsi qu'évacués sur un dispositif d'éjection (12), **caractérisé en ce qu'**une première zone de traitement est prévue pour mouiller le matériel infecté, en créant dans une première zone de la chambre de traitement (6), par une inclinaison dirigée vers le haut dans le sens de transport essentiellement de la totalité de la chambre de traitement (6), un réservoir de liquide (16) au moyen de liquide présent dans le matériel infecté et/ou d'addition externe d'eau, et le réservoir de liquide est chauffé à une température inférieure à la température d'ébullition de l'eau, ladite première zone étant voisine de l'extrémité située plus bas de la chambre de traitement,
et une deuxième zone de traitement est prévue pour la désinfection ou la stérilisation en chauffant une deuxième zone qui s'étend de la première zone jusqu'à l'extrémité située plus haut de la chambre de traitement (6) au moins partiellement à une température supérieure à la température d'ébullition de l'eau, et en créant dans la deuxième zone la pression de vapeur nécessaire à la désinfection ou à la stérilisation.

2. Procédé selon la revendication 1, **caractérisé en ce que** la deuxième zone est subdivisée en sections de température différente.

3. Procédé selon la revendication 2, **caractérisé en ce que**, dans la deuxième zone, la pression de vapeur voulue est produite par évaporation de l'humidité propre du matériel à traiter et/ou par amenée d'eau sous forme liquide et/ou de vapeur d'eau.

4. Procédé selon une des revendications précédentes, **caractérisé en ce que** le niveau du réservoir de liquide (16) est régulé au moyen d'un trop-plein (15).

5. Procédé selon la revendication 4, **caractérisé en ce que** du liquide est ramené du trop-plein (15) dans le réservoir de liquide (16).

6. Procédé selon l'une des revendications précédentes, **caractérisé en ce que** le matériel à traiter est amené en portions qui se trouvent en même temps dans la chambre de traitement (6), l'amenée et l'évacuation étant effectuées par l'intermédiaire de vannes ou de sas de l'unité d'entrée (2, 3, 4) et du. dispositif d'éjection (12).

7. Procédé selon l'une des revendications précédentes, **caractérisé en ce que** le système de transport présente un transporteur à vis (9).

8. Dispositif pour le traitement de matériels contaminés, en particulier infectés, dans lequel ceux-ci sont amenés par l'intermédiaire d'une unité d'entrée (2, 3, 4) à un système de transport (8) s'étendant dans une chambre de traitement (6), chauffés et, là, désinfectés ou stérilisés, ainsi qu'évacués sur un dispositif d'éjection (12), la totalité de la chambre de traitement (6) essentiellement étant inclinée vers le haut dans le sens du transport et la chambre de traitement (6) présentant une première zone de chauffage voisine de l'extrémité située plus bas de la chambre de traitement et une deuxième zone de chauffage qui s'étend entre la première zone de chauffage et la zone située plus haut de la chambre de traitement (6) et qui est conçue pour produire une température permettant d'obtenir la pression de vapeur nécessaire à la désinfection ou à la stérilisation, **caractérisé en ce que** la première zone de chauffage est équipée de moyens (23, réservoir de liquide 16) pour mouiller de liquide les matériels introduits dedans et pour produire une température située au-dessous de la température d'ébullition de l'eau et la deuxième zone de chauffage est conçue pour produire une température située au-dessus de la température d'ébullition de l'eau.

9. Dispositif selon la revendication 8, **caractérisé en ce que** la deuxième zone de chauffage est subdivisée en d'autres sections de chauffage pour produire d'autres températures.

10. Dispositif selon la revendication 8 ou 9, **caractérisé en ce que** la deuxième zone de chauffage présente des moyens (23) pour introduire de la vapeur d'eau.

11. Dispositif selon les revendications 8 à 10, **caractérisé en ce que** la première zone de chauffage présente des moyens (23) pour ajouter de l'eau liquide.

12. Dispositif selon l'une des revendications 8 à 11, **caractérisé en ce que** la chambre de traitement (6) présente un trop-plein (15) pour réguler un réservoir de liquide (15).

13. Dispositif selon la revendication 12, **caractérisé en ce que** le trop-plein (15) débouche dans un réservoir récepteur (18) qui est relié avec la chambre de traitement (6) par une tuyauterie de retour (19).

14. Dispositif selon la revendication 13, **caractérisé en ce que** le trop-plein (15), le réservoir récepteur (18) et la tuyauterie de retour (19) sont conçus pour qu'il y règne la même pression que dans la chambre de traitement (6).

15. Dispositif selon l'une des revendications 8 à 14, **caractérisé en ce que** la paroi de la chambre de traitement (6) est munie de moyens de chauffage.

16. Dispositif selon l'une des revendications 8 à 15, **caractérisé en ce que** le système de transport (9) est muni de moyens de chauffage.

17. Dispositif selon l'une des revendications 8 à 16, **caractérisé en ce que** de l'énergie par micro-ondes peut être introduite de manière définie dans la chambre de traitement (6) et/ou le système de transport (9).

18. Dispositif selon l'une des revendications 8 à 17, **caractérisé en ce que** le système de transport présente un transporteur à vis (9).

19. Dispositif selon la revendication 18, **caractérisé en ce que** le transporteur à vis (9) présente un palier à une extrémité et repose sur des rails d'usure.

20. Dispositif selon l'une des revendications 8 à 19, **caractérisé en ce qu'**un broyeur (3) est disposé dans l'unité d'entrée.

21. Dispositif selon l'une des revendications 8 à 20, **caractérisé en ce que** l'unité d'entrée (2, 3, 4) et le dispositif d'éjection (12) peuvent être fermés au moyen de vannes (7, 14) et/ou de sas.

22. Installation qui présente plusieurs dispositifs selon l'une des revendications 8 à 21 et une unité de broyage, les dispositifs étant disposés de manière parallèle de façon à pouvoir être alimentés par l'unité de broyage simultanément et/ou de manière décalée dans le temps.
